# EUROPEAN PATENT APPLICATION

(11) **EP 2 666 407 A1**
(43) Date of publication of application: **27.11.2013**
(21) Application number: 12173017.0
(22) Date of filing: 21.06.2012
(51) Int. Cl.: A61B 5/02

(54) **Outside clinical monitoring apparatus for monitoring a person**

(30) Priority: 22.05.2012 US 201261649968 P
(71) Applicant: Koninklijke Philips N.V., 5656 AE Eindhoven (NL)
(72) Inventor: The designation of the inventor has not yet been filed
(74) Representative: Jungen, Carmen

(57) **Abstract**

The invention relates to an outside clinical monitoring apparatus (1) for monitoring a person (6). The outside clinical monitoring apparatus is adapted to monitor a risk of a person being indicative of the likelihood that the person becomes ill. Clinical medical data of the person, which are required for determining the risk, are measured in a clinic (7), and outside clinical medical data are measured outside the clinic, wherein the measurements performed outside the clinic can be technically relatively simple and form a subset of the measurements performed in the clinic. The outside clinical monitoring apparatus (1) determines the risk based on the outside clinical medical data of the subset of measurements and based on the clinical medical data of the further measurements. The outside clinical monitoring apparatus can therefore be used to monitor the risk in a technically relatively simple way outside the clinic, in particular, at home (5).

## Description

### FIELD OF THE INVENTION

The invention relates to an outside clinical monitoring apparatus, an outside clinical monitoring method and an outside clinical monitoring computer program for monitoring a person.

### BACKGROUND OF THE INVENTION

US 2010/0081941 A1 discloses the calculation of a risk score based on the results of several clinical measurements performed in a clinic. These clinical measurements include, for instance, magnetic resonance imaging, computed tomography imaging, coronary calcium scoring, et cetera, are technically very complex and need professional assistance. For monitoring the risk score over time, these technically complex measurements have to be performed several times, thereby rendering the monitoring of the risk score technically very complex.

### SUMMARY OF THE INVENTION

It is an object of the present invention to provide an outside clinical monitoring apparatus, an outside clinical monitoring method and an outside clinical monitoring computer program for monitoring a person, which allow monitoring the person in a technically less complex way.

In a first aspect of the present invention an outside clinical monitoring apparatus for monitoring a person is presented, wherein the outside clinical monitoring apparatus is adapted to monitor a risk of a person being indicative of the likelihood that the person becomes ill, the outside clinical monitoring apparatus comprising:
- a clinical medical data receiving unit for receiving clinical medical data of the person which are required for determining the risk, the clinical medical data having been measured in a clinic,
- an outside clinical medical data receiving unit for receiving outside clinical medical data of the person, the outside clinical medical data resulting from measurements outside the clinic forming a subset of measurements performed in the clinic for providing the clinical medical data,
- a risk determination unit for determining the risk based on the received outside clinical medical data of the subset of measurements and based on the received clinical medical data of the further measurements.

Since the clinical medical data receiving unit receives the clinical medical data of the person which are required for determining the risk, wherein the outside clinical medical data receiving unit receives outside clinical medical data of the person at the outside of the clinic by performing measurements outside the clinic forming a subset of measurements performed in the clinic, a subset of the clinical medical data, which have been measured in the clinic, can be updated by using the outside clinical medical data, wherein the risk determination unit can determine the risk based on the updated data, which have been measured outside the clinic, and the clinical medical data, which correspond to the further measurements performed in the clinic. The measurements, which are performed outside the clinic for updating the initial clinical medical data, can be technically relatively simple measurements, which do not require the technically complex measurement systems provided in the clinic. The risk can therefore be monitored over time just by performing the technically relatively simple measurements outside the clinic, thereby allowing monitoring the risk in a technically less complex way.

The clinical medical data receiving unit is preferentially adapted to receive all clinical medical data of the person which are required for determining the risk. The outside clinical monitoring apparatus is preferentially a mobile apparatus like a handheld apparatus, which may be used at home. Thus, the outside clinical monitoring apparatus can be regarded as being a home monitoring apparatus, which is adapted to monitor the risk of the person based on home medical data of the person received by a home medical data receiving unit for determining the risk.

The risk can indicate the individual's likelihood to get a disease, in particular, to experience an acute negative event. The risk determination unit is preferentially adapted to determine a risk score on a discrete or a continuous scale. The risk determination unit can also be adapted to determine a risk category, i.e. the risk determination unit can also be adapted to perform a risk stratification. In particular, the risk determination unit can be adapted to firstly determine a risk score and to then assign the person to a risk category based on the determined risk score.

The risk determination unit can be adapted to determine a cardiovascular disease (CVD) risk score based on the clinical medical data and the outside clinical medical data.

In particular, the risk determination unit can be adapted to determine at least one of the Framingham, the Reynolds, the Rasmunssen, the SCORE and the PROCAM risk score as the CVD risk score. The risk determination unit can be adapted to use the algorithm disclosed in the Journal of the American Medical Association, 2007; 297: 611-619 or the algorithm disclosed in the article Circulation, 2008; 118: 2243-2251 for determining the Reynolds risk score, to use the algorithm disclosed in the Journal of the American College of Cardiology, 2007; 50: 840-842 for determining the Rasmunssen risk score, to use the algorithm disclosed in the European Heart Journal, 2003; 24: 987-1003 for determining the SCORE risk score and to use the algorithm disclosed in the article Circulation, 2002; 105: 310-315 for determining the PROCAM risk score. The outside clinical monitoring apparatus can therefore monitor a high quality risk score like the Framingham, the Reynolds, the Rasmunssen, the SCORE , or the PROCAM risk score in a technically relatively simple way by just performing relatively simple medical measurements, which do not require technically complex clinical medical measurement systems.

The risk determination unit can also be adapted to perform a risk stratification, in order to determine a risk category for the person. In particular, the risk determination unit can be adapted to use the risk stratification algorithm disclosed in the Journal of the American College of Cardiology, doi:10.1016/j.jacc.2010.09.001 or the risk stratification algorithm disclosed in the European Heart Journal, doi:10.1093/eurheartj/ehs092, which stratify individuals into risk categories like low, medium and high risk categories.

It is preferred that the outside clinical medical data receiving unit is adapted to receive outside clinical medical data from a medical outside clinical measuring device for measuring outside clinical medical data of the person outside the clinic. The outside clinical data receiving unit can comprise an input unit for allowing the person to input the measured outside clinical medical data such that the outside clinical medical data receiving unit receives the outside clinical medical data. Or the outside clinical medical data receiving unit can be adapted to be connected via a wired or wireless connection to the medical outside clinical measuring device for receiving the outside clinical medical data from the medical outside clinical measuring device.

It is further preferred that the outside clinical measuring device is adapted to perform at least one of a blood pressure measurement, a cholesterol measurement, a triglycerides measurement, a hsCRP measurement, a HbA1c, a glucose measurement and a body mass index measurement. These measurements can be performed in a technically relatively simple way such that the risk can be monitored with reduced technical efforts based on these measurements.

It is also preferred that the clinical medical data receiving unit is adapted to receive the clinical medical data from a clinical data management system within the clinic and to locally store the received clinical medical data for providing them to the risk determination unit for allowing the risk determination unit to determine the risk. Thus, initially clinical medical data, which are required for determining the risk, can be received from a clinical data management system within the clinic, wherein these initial clinical medical data can then be updated by using the outside clinical medical data, which are measured outside the clinic in a technically relatively simple way, wherein the updated data can be used for determining an updated risk. This updating can be performed several times with relatively low technical efforts, because it is just required to perform the technically relatively simple outside clinical measurements, thereby monitoring the clinical risk in a technically relatively simple way over time.

The outside clinical monitoring apparatus preferentially further comprises a deviation determination unit for determining a deviation between the determined risk and at least one of a clinical risk, which has been determined based on the clinical medical data only, and a provided target risk. Moreover, the outside clinical monitoring apparatus preferentially further comprises an output unit for outputting the determined risk. The output unit is preferentially a display unit for displaying the determined risk. The output unit can also be adapted to output the determined deviation between the determined risk and the clinical risk or the target risk. Thus, an indication can be provided to the person for prompting the person, for instance, to talk to a physician and/or to modify, for instance, eating habits for improving, i.e. lowering, the risk.

The outside clinical medical data receiving unit can be adapted to receive outside clinical medical data of the person measured outside the clinic at different times, wherein the risk determination unit can be adapted to determine several risks for the different times depending on the received outside clinical medical data measured outside the clinic at different times and depending on the received clinical medical data, wherein the outside clinical monitoring apparatus further comprises a storing unit for storing the several risks corresponding to the different times. Thus, a log of risks can be maintained as a function of time, in order to chart the progress of the person.

In a further aspect of the present invention an outside clinical monitoring method for monitoring a person is presented, wherein the outside clinical monitoring method is adapted to monitor a risk of a person being indicative of the likelihood that the person becomes ill, the outside clinical monitoring method comprising:
- receiving clinical medical data of the person which are required for determining the risk by a clinical medical data receiving unit, the clinical medical data having been measured in a clinic,
- receiving outside clinical medical data of the person by performing measurements outside the clinic forming a subset of measurements by an outside clinical medical data receiving unit, the outside clinical medical data resulting from measurements outside the clinic forming a subset of measurements performed in the clinic for providing the clinical medical data,
- determining the risk based on the received outside clinical medical data of the subset of measurements and based on the received clinical medical data of the further measurements by a risk determination unit.

In another aspect of the present invention an outside clinical monitoring computer program for monitoring a person is presented, wherein the outside clinical monitoring computer program is adapted to monitor a risk of a person being indicative of the likelihood that the person becomes ill, the computer program comprises program code means for causing an outside clinical monitoring apparatus as defined in claim 1 to carry out the steps of the outside clinical monitoring method as defined in claim 10, when the computer program is run on a computer controlling the outside clinical monitoring apparatus.

It shall be understood that the outside clinical monitoring apparatus of claim 1, the outside clinical monitoring method of claim 10 and the outside clinical monitoring computer program of claim 11 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following drawings:
- Fig. 1: shows schematically and exemplarily a cooperation between devices in a clinic and devices at home,
- Fig. 2: shows schematically and exemplarily a home monitoring apparatus for monitoring a person, and
- Fig. 3: shows a flowchart exemplarily illustrating an embodiment of a home monitoring method for monitoring a person.

### DETAILED DESCRIPTION OF EMBODIMENTS

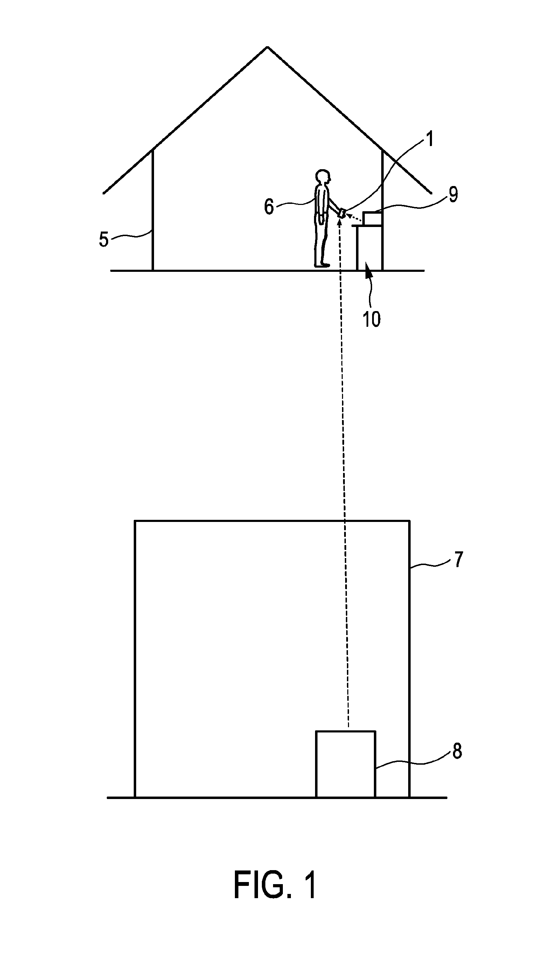

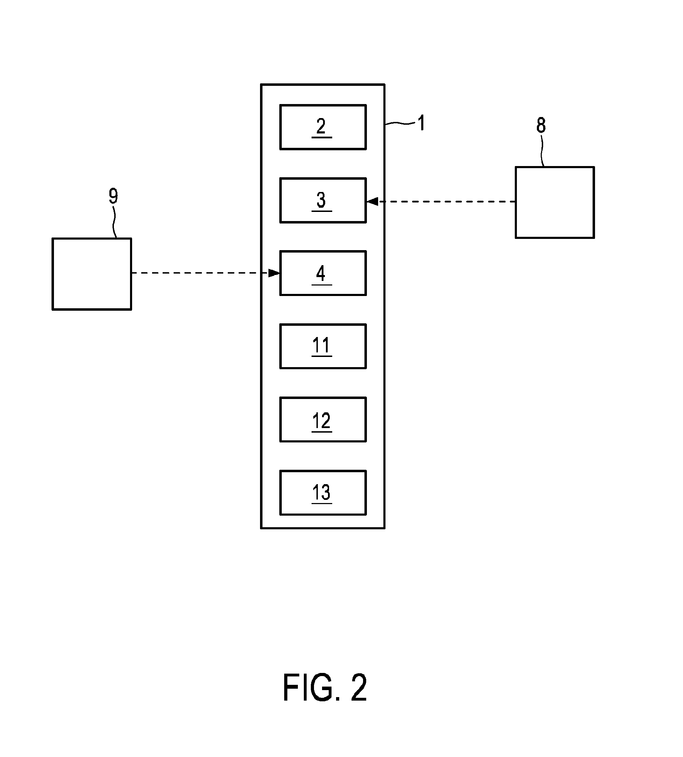

Fig. 1 shows schematically and exemplarily a person 6 within his/her home 5 holding a mobile outside clinical monitoring apparatus being, in this embodiment, a home monitoring apparatus 1. The home monitoring apparatus 1 is exemplarily and schematically shown in more detail in Fig. 2.

In this embodiment, the home monitoring apparatus 1 is adapted to monitor a risk score of the person 6 being indicative of the likelihood that the person 6 becomes ill. In particular, the home monitoring apparatus 1 is adapted to monitor a CVD risk score. The home monitoring apparatus 1 comprises a clinical medical data receiving unit being, in this embodiment, a home medical data receiving unit 3 for receiving all clinical medical data of the person 6, which are required for determining the CVD risk score. The clinical medical data have been measured in a clinic 7 and are stored in a clinical data management system 8. The home medical data receiving unit 3 is adapted to receive the clinical medical data from the clinical data management system 8 within the clinic 7 and to locally store the received clinical medical data for providing them to a risk determination unit being, in this embodiment, a risk score determination unit 11 for allowing the risk score determination unit 11 to determine the risk score.

The home monitoring apparatus 1 further comprises an outside clinical medical data receiving unit being, in this embodiment, a home medical data receiving unit 4 for receiving home medical data of the person 6 at the home of the person 6 by performing measurements at home, which form a subset of the measurements performed in the clinic 7 for providing the clinical medical data stored in the clinical data management system 8 within the clinic 7. The home medical data receiving unit 4 is adapted to receiving home medical data from a medical outside clinical measuring device being, in this embodiment, a home measuring device 9 for measuring home medical data of the person 6 at the home of the person 6.

The home measuring device 9 can be adapted to perform at least one of a blood pressure measurement, a cholesterol measurement, a triglycerides measurement, a hsCRP measurement, glucose, a HbA1c measurement and a body mass index measurement. The home measuring device 9 can be adapted to allow performing these measurements in a known way.

The home data receiving unit 4 can be adapted to allow a user to input the measured home medical data such that the home medical data receiving unit 4 receives the same. Alternatively or in addition, the home medical data receiving unit 4 can be adapted to be connected via a wired or wireless connection with the medical home measuring device 9 for receiving the home medical data from the medical home measuring device 9.

The mobile home monitoring apparatus 1 further comprises the risk score determination unit 11 for determining the risk score based on the received home medical data of the subset of measurements and based on the received clinical medical data of the further clinical measurements, which have been performed in the clinic 7. In particular, the risk score determination unit 11 is adapted to determine at least one of the Framingham, the Reynolds, the Rasmunssen, SCORE and the PROCAM risk score as the CVD risk score.

The home monitoring apparatus 1 further comprises a deviation determination unit 12 for determining a deviation between the determined risk score and at least one of a clinical risk score, which can be determined based on the clinical medical data only, and a provided target risk score. The home monitoring apparatus 1 also comprises an output unit 2 being, in this embodiment, a display unit for displaying the determined risk score and the determined deviation, in order to show the determined risk score and the deviation to the person 6.

The home medical data receiving unit 4 is preferentially adapted to receive the home medical data of the person 6 measured at the home 5 at different times, i.e. the home medical data can be measured by the home measuring device 9, which, in this embodiment, is located on a support 10 being, for instance, a table or a desk, at the different times, wherein these home medical data can be provided to the home medical data receiving unit 4. Moreover, the risk score determination unit 11 is preferentially adapted to determine several risk scores for the different times depending on the received home medical data measured at the home 5 at the different times and depending on the received clinical medical data, wherein these risk scores can be stored in a storing unit 13. Thus, a log of risk scores can be maintained as a function of time, in order to chart the progress of the person. The progress of the person can be shown on the display unit 2.

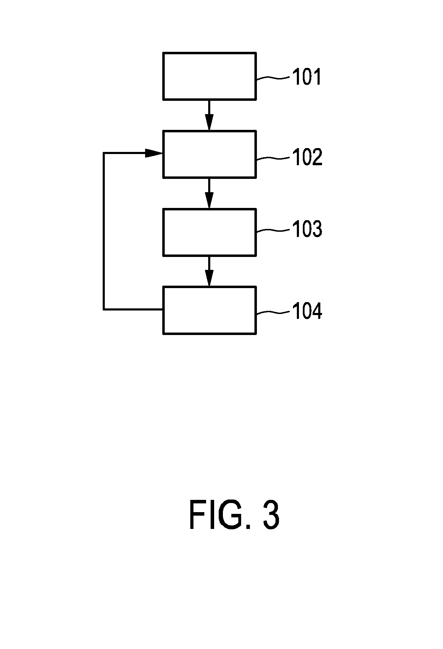

In the following an embodiment of an outside clinical monitoring method for monitoring a person will exemplarily be described with reference to a flowchart shown in Fig. 3.

In step 101 all clinical medical data of the person, which are required for determining the risk score, are received by the clinical medical data receiving unit. In particular, all measurements needed for determining the risk score have been performed in the clinic and stored in the clinical data management system. From this clinical data management system the clinical medical data receiving unit preferentially receives all clinical medical data of the person, which are required for determining the risk score. In step 102 home medical data of the person are received by performing measurements at the home of the person forming a subset of measurements, which are performed in the clinic for providing the clinical medical data, by the home medical data receiving unit. The subset of measurements includes measurements, which can be performed by the person at home and which measure properties of the person that may change with time. In step 103 the risk score is determined based on the received home medical data of the subset of measurements and based on the received clinical medical data of the further measurements by the risk score determination unit. In step 104 the determined risk score is stored in the storing unit. The home monitoring method can repeat steps 102 to 104 several times for monitoring the risk score and for storing the monitored risk score over time.

The home monitoring apparatus is preferentially a device that enables home monitoring of the risk profile of individuals, after a full risk assessment has been performed in the clinic. The device preferentially stores all the individual measurement/test results from the risk assessment in the clinic and recalculates the risk of the individual, i.e. of the person, preferentially on a regular basis outside the clinic. The recalculation can be based on new inputs using tests that the individual can perform outside a clinical setting, in particular, at home. Additional parameters needed for the recalculation, which cannot be measured by the individual himself, can be taken from the data of the previous clinic visit. In this way an individual can be shown how he/she is improving with respect to a target and the individual can get a feedback about the effectiveness of the individual complying or non-complying with clinicians' treatment recommendations.

CVD is one of the leading courses of death worldwide. Unlike other diseases CVD can be prevented often only with simple lifestyle modification measures. Although it is generally accepted that all individuals should aim at a healthy lifestyle, in order to prevent CVD, there are individuals that are at higher risk, in particular, in 10 years, of experiencing a severe CVD event such as heart attack, stroke or death. The home monitoring apparatus can be used to effectively identify individuals, who are vulnerable for a CVD event, with reduced technical complexity and single them out for medical treatment, lifestyle coaching and more healthcare attention.

The risk score calculated by the home monitoring apparatus can be used to determine whether an individual falls into a low, medium or high risk category. Based on the categories certain treatment options can be recommended. In particular, the home monitoring apparatus can comprise assignments between risk scores and categories such that based on the determined risk score and these assignments the individual can be assigned to a low risk category, a medium risk category or a high risk category. Moreover, the home monitoring apparatus can comprise assignments between these categories and measures for lowering the risk score, wherein based on the respective category and these assignments a corresponding measure can be recommended. The measure can be, for instance, a certain treatment, an interview with a clinician and/or a lifestyle modification. The risk scores can give an individual a quantitative indication of his health status by indicating the likelihood of experiencing an acute event in the future.

Treatment recommendations made by clinicians are aimed at reducing the risk as measured by the risk score of individuals. To determine whether the health of the individual has improved, i.e. whether the risk score has been lowered, after following the recommendations, generally an individual has to return to the clinic and undergo a full risk assessment, in order to recalculate the new risk score. Due to the technical effort acquired for such a risk assessment, these are not done at frequent intervals, for instance, they are only done yearly. Thus, it is generally difficult to monitor the individual's progress and give him regular feedback as to how the individual is progressing. In order to overcome this problem, the home monitoring apparatus is adapted to regularly determine an individual's risk score outside the clinic with only a few simple measurements that the individual can perform himself/herself. In this way the individual can see how the individual is progressing with respect to a target. Moreover, it may be that the individual has reached the target, but due to short-term changes the individual has deviated again, wherein the home monitoring apparatus is preferentially adapted to indicate this deviation as well.

The home monitoring apparatus is preferentially adapted to connect to a personal clinical profile on a clinical data management system and to locally store all test results from a previous clinical risk assessment. The home monitoring apparatus can preferentially regularly request and store results from new measurements performed by the individual at home. These tests are a subset of the measurements required for risk assessment. The home monitoring apparatus is preferentially further adapted to calculate a new risk score based on the previous test results from the clinic and the results from the subset of tests performed at home. The home monitoring apparatus can then preferentially be used as a risk improvement advisor which shows the new risk score relative to the previous risk score, which is determined based on the clinical measurements, and relative to a target risk score.

Based on the risk score individuals can be separated into low, intermediate and high categories. For instance, first the Framingham risk score calculations can be used and, if the risk score is smaller than 6 percent, the individuals can be assigned to the low category. If the risk score is between 6 and 20 percent, the individuals can be assigned to the intermediate category, and if the risk score is equal to or larger than 20 percent, the individuals can be assigned to the high category. In the clinic additional tests can be performed for the intermediate category, in order to reclassify the individuals into low or high, if possible. These additional clinical tests can include ankle-brachial index, carotid intima media thickness, plaque presence and coronary calcium scoring tests. For individuals having diabetes and/or hypertension additionally values from a testing of HbA1c and microalbuminuria can be included in the risk score calculation or in the category reclassification. These tests are preferentially all performed in the clinic for determining the risk score.

Generally depending on the category, to which the individual has been assigned, the individual is asked to return to the clinic for a follow-up risk assessment in six to twelve months. Certain physiological processes can be reversed through treatment and tests can be performed to show these. For example, total cholesterol, HDL cholesterol, systolic blood pressure, ankle-brachial index, carotid intima media thickness and plaque presence can be measured dynamically to potentially show the progression/regression of the CVD risk score. The treatments can include cholesterol or blood pressure lowering medication and lifestyle modifications like diet, exercise, stress reduction, et cetera.

All of these test measurements necessary for a full assessment, i.e. necessary for determining the risk score, are initially performed in the clinic. Moreover, in the clinic the risk score is calculated and the individual is classified in accordance with the risk score. Moreover, it can be agreed upon a target risk score and to a treatment routine. The home monitoring apparatus is preferentially adapted to receive all the individual values of the tests, the score, the category and the target risk score, wherein this transfer of the measurement results can be performed in a wired or wireless way in the clinic or, for instance, via the Internet.

The home monitoring apparatus can be adapted to ask the respective person to perform measurements regularly, for instance, in the time scale of weeks, regarding a subset of parameters in the risk score. For instance, if the risk score is the Framingham risk score, blood pressure, smoking status and body mass index data can be received by the home medical data receiving unit of the home monitoring apparatus, in order to allow the risk score determination unit of the home monitoring apparatus to update the risk score based on these home data and the clinical test results. The home measuring device can therefore comprise a blood pressure measurement unit for measuring the blood pressure of the person. Moreover, the home measuring device can also comprise home blood test kits for measuring the cholesterol value at home, wherein also this cholesterol value can be used for updating the risk score. The subset of parameters formed by blood pressure, cholesterol and smoking status can also be used for updating other scores like the SCORE, the PROCAM and the Reynolds scores. If the risk score determination unit is adapted to determine the PROCAM score, the home medical data receiving unit can be adapted to additionally receive values from home measurements of triglycerides and, if the risk score determination unit is adapted to determine the Reynolds risk score, the home medical data receiving unit is preferentially adapted to also receive values from home measurements of hsCRP and HbA1c. Thus, the medical home measuring device can be adapted to perform corresponding home blood tests, wherein the blood test results are provided to the home medical data receiving unit of the home monitoring apparatus, in order to allow the risk score determination unit to determine the respective risk score based on this subset of parameters and the clinical parameters for which values have been measured in the clinic.

The home medical data receiving unit can comprise an input unit like a keyboard, a touch screen, et cetera, in order to allow the individual to input the values, which have been measured at home, into the home medical data receiving unit of the home monitoring apparatus. Alternatively or in addition, a data connection between the home medical data receiving unit and the medical home measuring device, which may be wired or wireless, can be used for transferring home measurement values to the home medical data receiving unit. The risk score determination unit of the home monitoring apparatus can then recalculate the risk score using the original test values from the clinic and the updates for only the subset. The home monitoring apparatus can be adapted to compare the updated new risk score with the target risk score, in order to provide a feedback to the individual on how the individual is doing with respect to the target risk score. The home monitoring apparatus is preferentially also adapted to maintain a log of all the risk scores/categories as a function of time and charts the progress of the individual.

The home monitoring apparatus allows individuals and family physicians to monitor the risk score in a technically simple way with technically simple tests that individuals can perform themselves.

Although in the above described embodiments the outside clinical monitoring apparatus is a home monitoring apparatus, the monitoring apparatus can also be used at other locations outside the clinic, for instance, in a hotel, in a nursing home, et cetera.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Determinations like the determination of a risk score, the determination of a category, the determination of a deviation of an actual risk score from a target risk score or from a previously determined risk score, et cetera performed by one or several units or devices can be performed by any other number of units or devices. The determinations and/or the control of the outside clinical monitoring apparatus in accordance with the outside clinical monitoring method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an outside clinical monitoring apparatus for monitoring a person. The outside clinical monitoring apparatus is adapted to monitor a risk of a person being indicative of the likelihood that the person becomes ill. Clinical medical data of the person, which are required for determining the risk, are measured in a clinic, and outside clinical medical data are measured outside the clinic, wherein the measurements performed outside the clinic can be technically relatively simple and form a subset of the measurements performed in the clinic. The outside clinical monitoring apparatus determines the risk based on the outside clinical medical data of the subset of measurements and based on the clinical medical data of the further measurements. The outside clinical monitoring apparatus can therefore be used to monitor the risk in a technically relatively simple way outside the clinic, in particular, at home.

## Claims

1. An outside clinical monitoring apparatus for monitoring a person, the outside clinical monitoring apparatus being adapted to monitor a risk of a person being indicative of the likelihood that the person becomes ill, the outside clinical monitoring apparatus (1) comprising:
- a clinical medical data receiving unit (3) for receiving clinical medical data of the person which are required for determining the risk, the clinical medical data having been measured in a clinic (7),
- an outside clinical medical data receiving unit (4) for receiving outside clinical medical data of the person (6), the outside clinical medical data resulting from measurements outside the clinic forming a subset of measurements performed in the clinic for providing the clinical medical data,
- a risk determination unit (11) for determining the risk based on the received outside clinical medical data of the subset of measurements and based on the received clinical medical data of the further measurements.

2. The outside clinical monitoring apparatus as defined in claim 1,
wherein the risk determination unit (11) is adapted to determine a cardiovascular disease risk score based on the clinical medical data and the outside clinical medical data.

3. The outside clinical monitoring apparatus as defined in claim 2,
wherein the risk determination unit (11) is adapted to determine at least one of the Framingham, the Reynolds, the Rasmunssen, the SCORE and the PROCAM risk score as the cardiovascular disease risk score.

4. The outside clinical monitoring apparatus as defined in claim 1,
wherein the outside clinical medical data receiving unit (4) is adapted to receive outside clinical medical data from a medical outside clinical measuring device (9) for measuring outside clinical medical data of the person outside the clinic.

5. The outside clinical monitoring apparatus as defined in claim 4,
wherein the outside clinical measuring device (9) is adapted to perform at least one of a blood pressure measurement, a cholesterol measurement, a triglycerides measurement, a hsCRP measurement, glucose, a HbA1c measurement and a body mass index measurement.

6. The outside clinical monitoring apparatus as defined in claim 1,
wherein the clinical medical data receiving unit (3) is adapted to receive the clinical medical data from a clinical data management system (8) within the clinic (7) and to locally store the received clinical medical data for providing them to the risk determination unit (11) for allowing the risk determination unit (11) to determine the risk.

7. The outside clinical monitoring apparatus as defined in claim 1,
wherein the outside clinical monitoring apparatus (1) further comprises a deviation determination unit (12) for determining a deviation between the determined risk and at least one of a clinical risk, which has been determined based on the clinical medical data only, and a provided target risk.

8. The outside clinical monitoring apparatus as defined in claim 1,
wherein the outside clinical monitoring apparatus (1) further comprises an output unit (2) for outputting the determined risk.

9. The outside clinical monitoring apparatus as defined in claim 1,
wherein the outside clinical medical data receiving unit (4) is adapted to receive outside clinical medical data of the person measured outside the clinic at different times, wherein the risk determination unit (11) is adapted to determine several risks for the different times depending on the received outside clinical medical data measured outside the clinic at different times and depending on the received clinical medical data, wherein the outside clinical monitoring apparatus (1) further comprises a storing unit (13) for storing the several risks corresponding to the different times.

10. An outside clinical monitoring method for monitoring a person, the outside clinical monitoring method being adapted to monitor a risk of a person being indicative of the likelihood that the person becomes ill, the outside clinical monitoring method comprising:
- receiving clinical medical data of the person which are required for determining the risk by a clinical medical data receiving unit, the clinical medical data having been measured in a clinic,
- receiving outside clinical medical data of the person by performing measurements outside the clinic forming a subset of measurements by an outside clinical medical data receiving unit, the outside clinical medical data resulting from measurements outside the clinic forming a subset of measurements performed in the clinic for providing the clinical medical data,
- determining the risk based on the received outside clinical medical data of the subset of measurements and based on the received clinical medical data of the further measurements by a risk determination unit.

11. An outside clinical monitoring computer program for monitoring a person, the outside clinical monitoring computer program being adapted to monitor a risk of a person being indicative of the likelihood that the person becomes ill, the computer program comprising program code means for causing an outside clinical monitoring apparatus as defined in claim 1 to carry out the steps of the outside clinical monitoring method as defined in claim 10, when the computer program is run on a computer controlling the outside clinical monitoring apparatus.
